# EUROPEAN PATENT APPLICATION

(11) **EP 3 738 649 A1**
(43) Date of publication of application: **18.11.2020**
(21) Application number: 19187483.3
(22) Date of filing: 22.07.2019
(51) Int. Cl.: A61N 1/375, A61N 1/372

(54) **IMPLANTABLE MEDICAL DEVICE COMPRISING AN ANCHORING DEVICE**

(30) Priority: 16.05.2019 US 201916414060
(71) Applicant: BIOTRONIK SE & Co. KG, 12359 Berlin (DE)
(72) Inventor: Hughes, Devan, Tualatin, OR 97062 (US); Whittington, R. Hollis, Portland, OR 97202 (US); Muessig, Dirk, West Linn, OR 97068 (US); Kibler, Andrew B., Lake Oswego, OR 97035 (US); Suwito, Wantjinarjo, West Linn, OR 97068 (US); Austin, Eric, Portland, OR 97221 (US)
(74) Representative: Galander, Marcus

(57) **Abstract**

An implantable medical device (1) comprises a housing (10) having a proximal end (101) and a distal end (100), an electrode device (11) arranged in the region of the distal end (100), and an anchoring device (2) fixedly attached to the housing (10) in the region of the distal end (100). The anchoring device (2) comprises at least one anchoring member (20) having a preshaped first configuration, the at least one anchoring member (20) being deflectable from the preshaped first configuration into a strained second configuration for placement of the implantable medical device (1) on an object. The at least one anchoring member (20) comprises a tip section (21) and a connection section (22) extending in between the tip section (21) and the distal end (100) of the housing (10), wherein at least in the preshaped first configuration the tip section (21) comprises a straight portion (212) adjoining the connection section (22) at a transition location (211) by forming a bend in between the straight portion (212) and the connection section (22).

## Description

The invention generally relates to an implantable medical device and a delivery arrangement comprising a delivery system for delivering such an implantable medical device.

An implantable medical device of this kind may for example be a leadless pacemaker for implantation into the human heart, for example in the right ventricle or the right atrium.

In recent years, leadless pacemakers have received increasing attention. Leadless pacemakers, in contrast to pacemakers implanted subcutaneously using leads extending transvenously into the heart, avoid the need for leads in that the pacemaker device itself is implanted into the heart, the pacemaker having the shape of a generally cylindrical capsule for implantation into cardiac tissue, in particular the right ventricular wall of the right ventricle. Such leadless pacemakers exhibit the inherent advantage of not using leads, which can reduce risks for the patient involved with leads transvenously accessing the heart, such as the risk of pneumothorax, lead dislodgement, cardiac perforation, venous thrombosis and the like.

When placing an implantable medical device such as a leadless pacemaker device for example in the human heart, one challenge lies in designing an anchoring device allowing a fixation of the implantable medical device for example on cardiac tissue such that the implantable medical device is securely received and held on cardiac tissue. For this, the anchoring device on the one hand shall provide for a secure fastening of the implantable medical device on the cardiac tissue, on the other hand however shall avoid a damaging of cardiac tissue and structures within the cardiac tissue.

A typical anchoring device comprises anchoring members in the form of tines which, when placing the implantable medical device on cardiac tissue, pierce the cardiac tissue and in this way provide for an anchoring of the implantable medical device on the cardiac tissue. For placing the implantable medical device on the cardiac tissue, the implantable medical device typically is received within a sheathing device of a delivery catheter, the delivery catheter being used to access the human heart and to deliver the implantable medical device towards a location of interest by removing the sheathing device and by in this way placing the implantable medical device on cardiac tissue at the location of interest. When received in the sheathing device, anchoring members of the anchoring device are deformed from a relaxed, preshaped first configuration to assume a strained second configuration within the sheathing device. The anchoring members reset to their preshaped first configuration upon delivering the implantable medical device from the sheathing device such that the anchoring members engage with cardiac tissue to fasten the implantable medical device to the cardiac tissue.

There is a general desire to provide for an anchoring device that allows an easy, reliable delivery from a sheathing device, while providing for a secure and reliable fastening when placing the implantable medical device on an object, in particular on cardiac tissue.

Document US 9,844,659 B2 describes an assembly of an implantable medical device comprising an anchoring device having a set of active fixation tines. The active fixation tines in the set are deployable from a spring-loaded position in which distal ends of the fixation tines point away from the implantable medical device to a hooked position in which the active fixation tines bend back towards the implantable medical device.

Document US 2015/0051616 A1 discloses an implantable leadless pacing device including a pacing capsule having a housing. Anchoring members are coupled to a distal region of the housing, the anchoring members including a region with a compound curve.

It is an object to provide an implantable medical device and a delivery arrangement for delivering such implantable medical device which allow for a reliable and secure delivery of an implantable medical device to a location of interest for example in the human heart and a reliable fastening of the implantable medical device at a location of interest, for example to cardiac tissue.

An implantable medical device according to claim 1 and a delivery arrangement according to claim 11 are provided. Further embodiments are subject matter of dependent claims.

This object is addressed by an implantable medical device comprising a housing having a proximal end and a distal end, an electrode device arranged in the region of the distal end, and an anchoring device fixedly attached to the housing in the region of the distal end. The anchoring device comprises at least one anchoring member having a preshaped first configuration, the at least one anchoring member being deflectable from the preshaped first configuration into a strained second configuration (where it may be held within an implantation tool) for placement of the implantable medical device on an object. The at least one anchoring member comprises a tip section and a connection section extending in between the tip section and the distal end of the housing, wherein at least in the preshaped first configuration the tip section comprises a straight portion adjoining the connection section at a transition location by forming a bend (or a kink) in between the straight portion and the connection section.

The implantable medical device comprises an anchoring device configured to fasten the implantable medical device on an object, in particular cardiac tissue, upon placement of the implantable medical device for example in the human heart. An implantable medical device of this kind may for example be a leadless pacemaker device configured to provide a pacing action, the implantable medical device comprising an electrode device for emitting a simulation signal towards the cardiac tissue in order to stimulate cardiac activity.

The anchoring device comprises at least one anchoring member. The at least one anchoring member assumes a preshaped first configuration when it is in a relaxed state. From this preshaped first configuration the at least one anchoring member may be (elastically) deformed, in particular for receiving the implantable medical device in a sheathing device of a delivery system. When received in a delivery system the at least one anchoring member hence is strained in a spring-loaded fashion and is held in said second configuration, wherein upon placement of the implantable medical device on an associated object, in particular on cardiac tissue of the human heart, and upon delivery out of the delivery system the at least one anchoring member elastically resets towards its preshaped first configuration, the at least one anchoring member thus piercing cardiac tissue as it returns to the relaxed, preshaped first configuration.

The at least one anchoring member within the delivery device assumes a predefined shape corresponding to the second configuration, which may be such that a scratching of a sheathing device by a free end of the at least one anchoring member is avoided when delivering the implantable medical device out of the sheathing device. For this, the at least one anchoring member comprises different sections, namely a tip section forming the far end of the at least one anchoring member and a connection section for connecting the tip section to the housing of the implantable medical device. The tip section comprises a straight portion adjoining the connection section at a transition location by forming a bend in between the straight portion and the connection section.

At its tip section the at least one anchoring member hence at least partially is formed straight. At a position in between the straight portion of the tip section and the connection section herein a bend is formed of small radius. In this way it is possible to shape the tip section such that it, with its free end, does not scratch on an inner wall of a sheathing device upon delivering the implantable medical device out of the sheathing device for placing the implantable medical device on an object, in particular on cardiac tissue of the human heart. Because a scratching of the sheathing device is avoided, a risk of releasing material of the delivery catheter potentially into the bloodstream of a patient is reduced, reducing the danger of potentially hazardous effects such as a risk for thrombi and/or lung emboli. In one embodiment, the connection of the tip section to the connection section may be discontinuous in that no smooth transition from the straight portion of the tip section to the connection section is formed, but the tip section is joined to the connection section at a sharp-angled kink.

In addition, because the tip section at least partially is formed straight, the tip section may be shaped such that it may engage with tissue at an advantageous angle, in particular at substantially 90°, when placing the implantable medical device on the tissue, hence allowing for an initially deep penetration of the tissue at a substantially perpendicular penetration direction, which may allow the use of an anchoring member of comparatively short length. In particular, the tip section initially penetrates into the tissue at a substantially perpendicular angle, upon which it transitions from its (spring-loaded) strained second configuration into the relaxed preshaped first configuration and in this way pierces through the tissue and exits from the tissue by means of the tip section to form a hook-like connection for the implantable medical device on the tissue. Because the at least one anchoring member may have a rather large curvature at its connection section the overall length of the anchoring member may be comparatively short.

In one embodiment, the connection section of the at least one anchoring member, at least in the preshaped first configuration, comprises at least one of at least one curved portion and at least one straight portion. The connection section hence may have a generally curved shape, wherein the connection section may be formed by a single curved portion or multiple different portions which adjoin each other and which may have a curved or straight shape.

In one embodiment, the connection section may be formed by a single curved portion having a single, constant radius of curvature.

In another embodiment, the connection section may be formed by a single curved portion adjoined by a single straight portion, the straight portion of the connection section for example forming the transition location with the straight portion of the tip section.

In another embodiment, the connection section may be formed by multiple curved portions. For example, the connection section may be formed by multiple different curved portions, the curved portions adjoining each other and having different radii of curvature. In another example, the connection section may be formed by curved portions in between which straight portions are arranged, the curved portions each having the same radius of curvature or different radii of curvature.

In yet another embodiment, the connection section may be formed by a first curved portion having a first curvature and a second curved portion having a second curvature opposite to the first curvature. The different curved portions hence are curved into different directions, wherein for example the second curved portion may form the transition location with the straight portion of the tip section.

The tip section may be formed entirely straight such that the tip section is made up by the straight portion. In yet another example the tip section may have a curved portion adjoining the straight portion, the curved portion for example forming the far end of the at least one anchoring member.

The tip section adjoins the connection section at a transition location by forming a bend in between the straight portion and the connection section. At least in the preshaped first configuration, herein, the straight portion of the tip section may form an angle with respect to a straight portion of the connection section extending from the transition location. Alternatively, the straight portion of the tip section may form an angle with a tangent to a curved portion of the connection section extending from the transition location. The angle herein may be in the range between 90° and 160°, beneficially in between 120° and 150°.

If the connection section adjoins the tip section by means of a curved portion, the angle is measured in between a tangent to the curved portion, the tangent being placed at the transition location such that it is tangential to the curved portion of the connection section. The angle to the straight portion of the tip section herein differs from 180° such that (in mathematical terms) a discontinuously differentiable connection in between the tip section and the connection section is formed at the transition location.

In one embodiment, the anchoring device comprises a ring element for fixing the anchoring device to the housing of the implantable medical device. The at least one anchoring member herein is fixed to the ring element, for example by forming the at least one anchoring member integrally with the ring element. The ring element provides for a fixation of the anchoring device on the housing of the implantable medical device such that the at least one anchoring member is held at the distal end of the housing of the implantable medical device.

The anchoring device may comprise a multiplicity of anchoring members, for example two, three, four or more anchoring members, each anchoring member having a preshaped first configuration and a strained second configuration and each anchoring member having a tip section and a connection section as described previously.

The at least one anchoring member may for example be made of a wire, a sheet, or a tube. The at least one anchoring member or the anchoring device as a whole may for example be made from a superelastic metal material, in particular a Nickel Titanium alloy, also denoted as Nitinol.

The object is also addressed by a delivery arrangement comprising a delivery system having a sheathing device, and an implantable medical device. The implantable medical device comprises a housing having a proximal end and a distal end, an electrode device arranged in the region of the distal end, and an anchoring device fixedly attached to the housing in the region of the distal end, the anchoring device comprising at least one anchoring member having a preshaped first configuration, the at least one anchoring member being deflectable from the preshaped first configuration into a strained second configuration for placement of the implantable medical device on an object. The at least one anchoring member herein comprises a tip section and a connection section extending in between the tip section and the distal end of the housing, wherein at least in the preshaped first configuration the tip section comprises a straight portion adjoining the connection section at a transition location by forming a bend in between the straight portion and the connection section. The at least one anchoring member, for placement of the implantable medical device on an object by means of delivery system, is received in the sheathing device of the delivery system in said strained second configuration.

For placement of the implantable medical device on an object, the implantable medical device is received within the sheathing system of the delivery system. For arranging the implantable medical device for example within the human heart the implantable medical device is guided through a venous access by means of the delivery system, in particular comprising a delivery catheter. Once a location of interest is reached, the implantable medical device is delivered from the sheathing device of the delivery system by withdrawing the sheathing device or by pushing the implantable medical device out of the sheathing system, such that the implantable medical device with its distal end comes to rest on tissue at the location of interest and by means of the anchoring device is fastened to the tissue.

Within the sheathing device the at least one anchoring member of the anchoring device of the implantable medical device is held in the strained second configuration, which is such that the at least one anchoring member by means of its tip section (which at least partially is formed flat) may engage with the tissue when delivering the implantable medical device out of the sheathing device. During the placement the at least one anchoring member resets from its strained second configuration to the relaxed, preshaped first configuration, in the course of which the at least one anchoring member penetrates through the tissue and exits from the tissue by means of its tip section, such that a hook connection in between the implantable medical device and the tissue is formed. In an implanted state the connection section extends through tissue such that by means of the connection section the implantable medical device is securely fastened to the tissue.

In one embodiment, the implantable medical device generally extends along a longitudinal axis, the implantable medical device for example having a generally cylindrical shape with the longitudinal axis forming a cylinder axis of the implantable medical device. The proximal end and the distal end of the implantable medical device herein are displaced with respect to each other along the longitudinal axis, wherein the implantable medical device is deliverable from the sheathing device for placement on said object in a placement direction aligned with the longitudinal axis. When delivering the implantable medical device from the sheathing device the implantable medical device hence, with its distal end, approaches associated tissue along the placement direction, causing the at least one anchoring member to engage with the tissue in order to fasten the implantable medical device on the tissue.

In the strained second configuration the straight portion of the tip section of the at least one anchoring member may, in one embodiment, extend substantially parallel to the longitudinal axis and may point in the placement direction. The at least one anchoring member is hence held within the sheathing device in its strained second configuration such that it points forward in the placement direction and hence may engage with the tissue at an angle of substantially 90°, which allows for a beneficial, deep penetration in particular at an initial penetration phase by means of the tip section. This may allow the use of anchoring members of rather short length, because the connection section may be formed with a rather large curvature, as the fixation of the implantable medical device may be achieved by a comparatively deep, but laterally short penetration (as compared to a shallow, but laterally spread-out penetration).

In one embodiment, in the preshaped first configuration the straight portion extends substantially parallel or at an obtuse angle with respect to the longitudinal axis. The straight portion herein may, in the preshaped first configuration, point in a direction generally opposite to the placement direction. In the relaxed, preshaped first configuration the tip section of the at least one anchoring member by means of its straight portion hence points away from the tissue such that by means of the connection section a hook-like connection of the anchoring member to the tissue is established. The connection section herein penetrates through the tissue and curves laterally outwards from the housing, such that the tip section exits from the tissue at a laterally remote location from the housing, the tip section being formed to extend parallel to the housing (i.e., parallel to the longitudinal axis) or at an obtuse angle with respect to the longitudinal axis such that the tip section with its free end points away from the housing.

The various features and advantages of the present invention may be more readily understood with reference to the following detailed description and the embodiments shown in the drawings. Herein,
- Fig. 1: shows a schematic view of the human heart;
- Fig. 2: shows a schematic drawing of an implantable medical device in the shape of a leadless pacemaker device, the implantable medical device having a housing and an anchoring device arranged thereon;
- Fig. 3: shows a schematic drawing of an implantable medical device received in a sheathing device of a delivery system;
- Fig. 4: shows a schematic drawing of an embodiment of an anchoring device;
- Fig. 5: shows a schematic drawing of an embodiment of an anchoring member of an anchoring device;
- Fig. 6: shows a schematic drawing of yet another embodiment of an anchoring member of an anchoring device;
- Fig. 7: shows a schematic drawing of a yet another embodiment of an anchoring member of an anchoring device; and
- Fig. 8: shows a schematic drawing of yet another embodiment of an anchoring member of an anchoring device.

Subsequently, embodiments shall be described in detail with reference to the drawings. In the drawings, like reference numerals designate like structural elements.
It is to be noted that the embodiments are not limiting for the present disclosure, but merely represent illustrative examples.

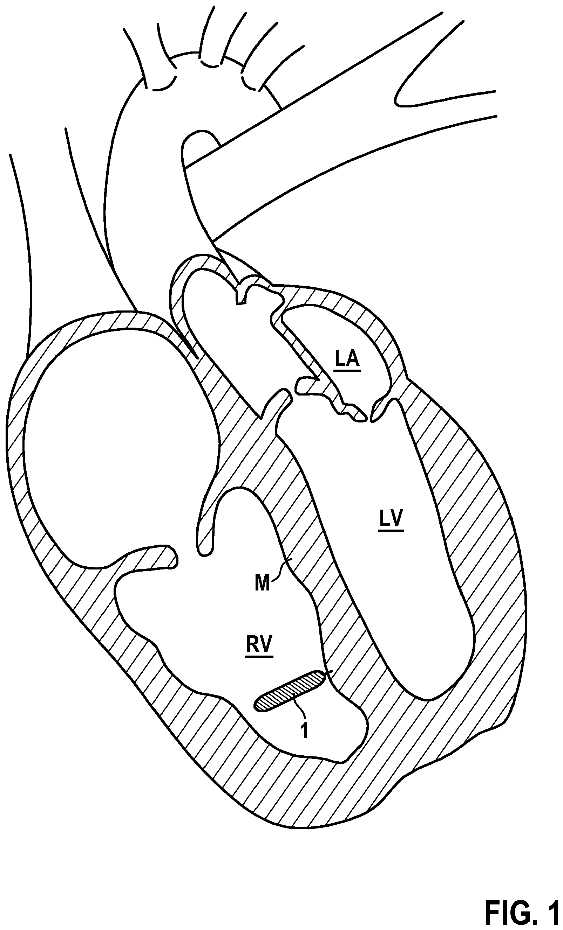

Fig. 1 shows, in a schematic drawing, a human heart comprising a right atrium RA, a right ventricle RV, a left atrium LA and a left ventricle LV, an implantable medical device 1 being implanted into the right ventricle RV, the implantable medical device 1 for example having the shape of a leadless pacemaker device for providing a pacing of the heart's activity at the right ventricle RV.

For implantation the implantable medical device 1 by means of a delivery system is placed in the right ventricle RV and is delivered from the delivery system such that it comes to rest on myocardial tissue M and is fastened to myocardial tissue M.

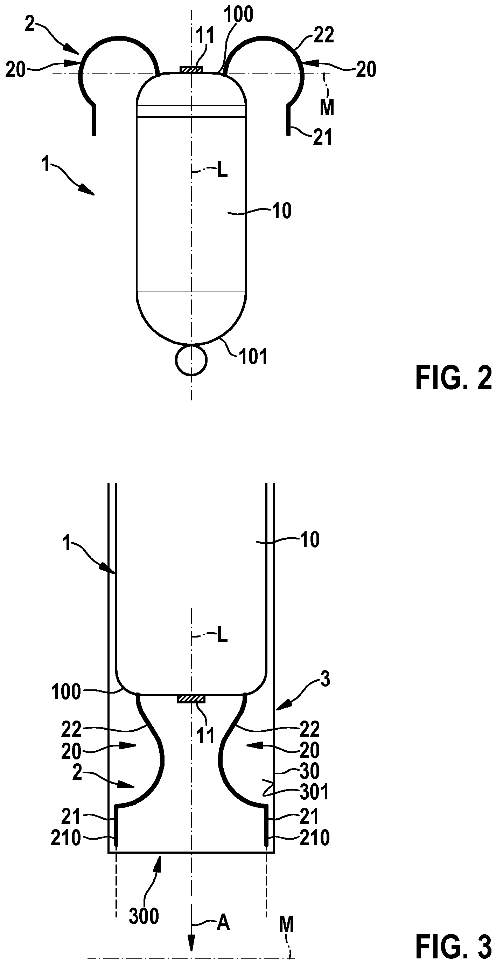

Fig. 2 shows, in a schematic drawing, an example of an implantable medical device 1 in the shape of a leadless pacemaker device, the implantable medical device 1 having a housing 10 forming a proximal end 101 and a distal end 100, the implantable medical device 1 being configured in its relaxed state, where the tines are in their preshaped first configuration. On the distal end 100, herein, an electrode device 11 is placed which, potentially together with other electrodes of the implantable medical device 1, serves to provide for a pacing action in order to stimulate cardiac activity in a defined manner.

During implantation the implantable medical device 1 is to be placed on myocardial tissue M and is to be fastened to the myocardial tissue M. For this, the implantable medical device 1 comprises an anchoring device 2 having a multiplicity of anchoring members 20 fabricated from wires, sheets or tubes or the like, the anchoring members 20 extending from the housing 10 of the implantable medical device 1 at the distal end 100 in order to engage with myocardial tissue M for securely fastening the implantable medical device 1 to the myocardial tissue M.

The anchoring members 20 are fixedly arranged on the distal end 100 of the housing 10 of the implantable medical device 1. In a relaxed state the anchoring members 20 assume a preshaped first configuration, as shown in Fig. 2, which is designed such that the anchoring members 20 may penetrate through myocardial tissue M in order to form hook-like connections in between the implantable medical device 1 and the myocardial tissue M. Each anchoring member 20 forms a tip section 21 and a connection section 22 extending in between the tip section 21 and the housing 10, the tip section 21 forming a free end of the respective anchoring member 20. In an implanted state, the tip section 21 may be placed outside of myocardial tissue M.

The anchoring members 20 of the anchoring device 2 may assume a strained second configuration in which they are deflected from their relaxed, preshaped first configuration, as it is schematically illustrated in Fig. 3. In particular, for placing the implantable medical device 1 on myocardial tissue M the implantable medical device 1 is received in a sheathing device 30 of a delivery system 3 in the shape of a delivery catheter, as illustrated in Fig. 3, the anchoring members 20 of the anchoring device 2 being held within the sheathing device 30 such that the tip sections 21 of the anchoring members 20 point forward in a placement direction A in which the implantable medical device 1 is to be placed on myocardial tissue M.

When having reached the myocardial tissue M at a point of interest the implantable medical device 1 is delivered from the sheathing device 30 in the placement direction A such that the implantable medical device 1 exits from the sheathing device 3 at a front opening 300, in the course of which the anchoring members 20 with their tip sections 21 engage with myocardial tissue M and penetrate into the myocardial tissue M.

As the anchoring members 20 are received within the sheathing device 30 in a spring-loaded, strained second configuration, the anchoring members 20, when the implantable medical device 1 is delivered from the sheathing device 30, relax from their second configuration towards the preshaped first configuration as illustrated in Fig. 2. In the course of the resetting movement the anchoring members 20 pierce through the myocardial tissue M and exit with their tip sections 21 from the myocardial tissue M, such that a hook-like connection of each anchoring member 20 with the myocardial tissue M is formed.

Whereas each anchoring member 20 at its tip section 21 is substantially formed straight, the connection section 22 of each anchoring member 20 has a substantially curved shape in order to form a loop through myocardial tissue M for fastening the implantable medical device 1 on the myocardial tissue M when the implantable medical device 1 is removed from the sheathing device 30 and the anchoring members 20 relax from their second configuration towards the preshaped first configuration.

Each anchoring member 20 herein generally has the same shape, wherein it also is conceivable that different anchoring members 20 of the anchoring device 2 have different shapes.

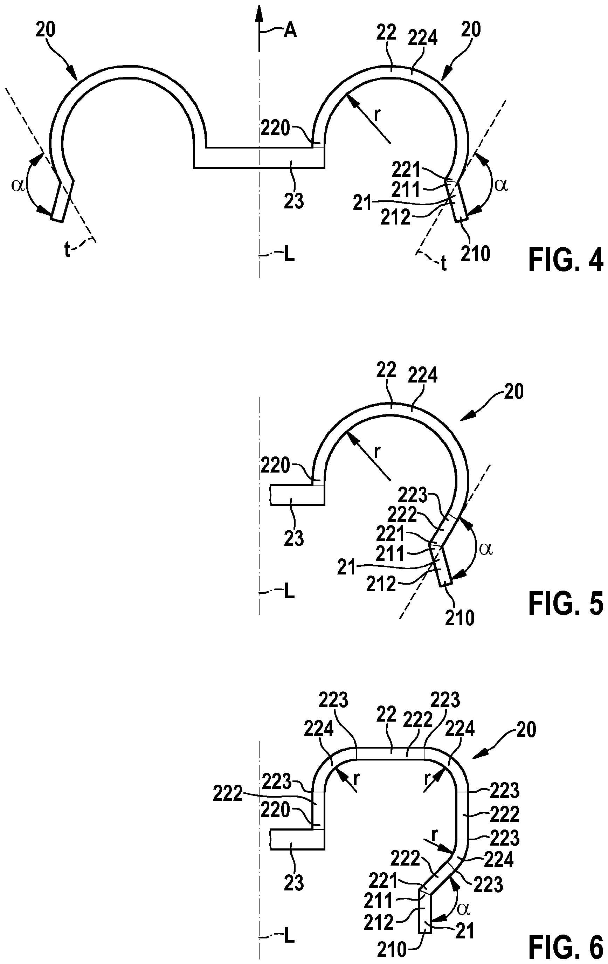

Different embodiments of anchoring members 20 of an anchoring device 2 are shown in Figs. 4 to 8.

The anchoring device 2 may, as illustrated in Fig. 4, comprise a ring element 23 which serves to fix the anchoring device 2 within the housing 10 and to fixedly hold the anchoring members 20 with respect to the housing 10. The ring element 23 may be received within the housing 10, the anchoring members 20 extending through the housing 10 at the distal end 100 such that the anchoring members 20 protrude from the distal end 100 as visible in Figs. 2 and 3.

The anchoring device 2 with its anchoring members 20 and the ring element 23 may be integrally formed for example from a superelastic Nickel Titanium alloy material. The anchoring members 20 herein are elastically deformable from their preshaped first configuration, which the anchoring members 20 assume when they are relaxed and are not received within the delivery system 3.

In Figs. 4 to 8 the anchoring members 20 are shown in the preshaped first configuration. In Figs. 5 to 8 herein only one anchoring member 20 is illustrated, wherein the anchoring device 2 may comprise one or multiple of such anchoring members 20.

In the embodiment of Fig. 4 each anchoring member 20 comprises a tip section 21 being formed by a straight portion 212 and a connection section 22 formed by a curved portion 224 having a (constant) radius of curvature r in the relaxed, preshaped first configuration as illustrated in Fig. 4. The connection section 22 adjoins the ring element 23 at an end 220. At an end 221 the connection section 22 is joined to the tip section 21, wherein the connection section 22 and the tip section 21 together form a bend at a transition location 211, the bend being defined by an angle α formed between the straight portion 212 of the tip section 21 and a tangent t to the curved portion 224 of the connection section 22 at the transition location 211.

In the embodiment of Fig. 4 the tip section 21 in the preshaped first configuration forms an obtuse angle to the longitudinal axis L along which the housing 10 of the implantable medical device 1 extends. The tip section 21 hence, in an implanted state of the medical device 1, comes to rest next to the housing 10 as visible from Fig. 2 and with its free end 210 points away from the housing 10.

In the embodiment of Fig. 5 the connection section 22 comprises a curved portion 224 having a (constant) radius of curvature r and a straight portion 222 adjoining the curved portion 224 at a transition location 223. The straight portion 222 is joined with the straight portion 212 of the tip section 21 at the transition location 211, the straight portion 222 of the connection section 22 and the straight portion 212 of the tip section 21 forming an angle α as visible from Fig. 5.

In the embodiment of Fig. 5 the tip section 21 with its straight portion 212 is arranged substantially in parallel to the longitudinal axis L of the housing 10 of the implantable medical device 1 when the anchoring member 20 is in its preshaped first configuration, as illustrated in Fig. 5.

In the embodiment of Fig. 6 the connection section 22 is formed by multiple curved portions 224, wherein in between neighboring curved portions 224 a respective straight portion 222 is formed. Each curved portion 224 has a similar radius of curvature r, the straight portions 222 and the curved portions 224 being connected with each other at transition locations 223.

In the embodiment of Fig. 6 the connection section 22 adjoins the ring element 23 and the tip section 21, at the transition location 211, by means of respective straight portions 222.

The tip section 21, in the embodiment of Fig. 6, is formed by a straight portion 212 and, in the relaxed, preshaped first configuration extends substantially in parallel to the longitudinal axis L of the housing 10.

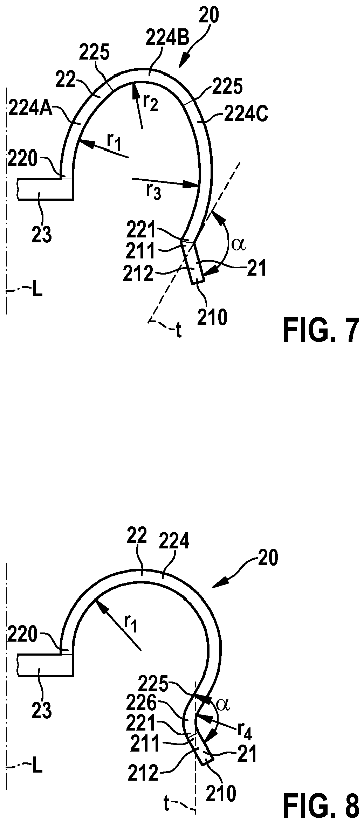

In the embodiment of Fig. 7 the connection section 22 is formed by different curved portions 224A, 224B, 224C, each curved portion 224A, 224B, 224C having a distinct radius of curvature r1, r2, r3. The curved portion 224A having a radius of curvature r1 joins the ring element 23, and the curved portion 224C having a radius of curvature r3 joins the tip section 21 at the transition location 211, an angle α being formed in between a tangent t to the curved portion 224C at the transition location 211 and the straight portion 212 of the tip section 21.

In the embodiment of Fig. 7 the tip section 210 with its straight portion 212 is arranged at an obtuse angle to the longitudinal axis L of the housing 10 in the relaxed, preshaped first configuration, the free end 210 of the tip section 21 pointing away from the housing 10.

In the embodiment of Fig. 8 the connection section 22 is formed by a first curved section 224 having a radius of curvature r1 and a second curved portion 226 having an opposite curvature with respect to the first curved portion 224 defined by a radius of curvature r4. The curved portion 226 joins the tip section 21 and the connection section 22, an angle α being formed in between a tangent t to the curved portion 226 at the transition location 211 and the straight portion 212 of the tip section 21.

In the embodiment of Fig. 8 the tip section 21 is arranged at an obtuse angle with respect to the longitudinal axis L of the housing 10 and, by means of its free end 210, points away from the housing 10.

The anchoring members 20 of the anchoring device 2 may, in particular, be shaped such that in the strained second configuration, as illustrated in Fig. 3, the anchoring members 20 with their free ends 210 do not scratch along an inner wall 301 of the sheathing device 30, but may bluntly about the inner wall 301, as illustrated in Fig. 3. Therefore the risk of particles entering the bloodstream due to scratching of the inner wall 301 of sheathing device 30 is reduced, which may help to reduce the risk for undesired effects such as thrombi and lung emboli.

As the straight portions 212 of the tip sections 21 of the anchoring members 20 point straight along the placement direction A when the implantable medical device 1 is received in the delivery system 3, the tip sections 21 may engage with myocardial tissue M at a substantially perpendicular angle, which may help to achieve a defined, deep penetration in particular at an initial phase of delivering the implantable medical device 1 from the sheathing device 30. This may allow to design the connection sections 22 with a relatively large curvature, such that in the implanted state (corresponding to the relaxed, preshaped first configuration of the anchoring members 20) the anchoring members 20 extend through the myocardial tissue M at a defined, narrow curve, allowing to form the anchoring members 20 with a comparatively small axial length.

In particular, the implantable medical device may be configured to act as a leadless pacemaker device, whereas the implantable medical device may also be designed to provide a different function. The implantable medical device may be configured to be implanted into the right ventricle of the human heart or into another location of, e.g., the human heart or another body part.

### List of Reference Numerals

- 1: Leadless pacemaker device
- 10: Housing
- 100: Distal end
- 101: Proximal end
- 11: Electrode
- 2: Anchoring device
- 20: Anchoring member
- 21: Tip section
- 210: End
- 211: Transition location
- 212: Straight portion
- 22: Connection section
- 220,221: End
- 222: Straight portion
- 223: Transition location
- 224: Curved portion
- 224A, B, C: Curved portion
- 225: Transition location
- 226: Curved portion
- 23: Ring element
- 3: Delivery system
- 30: Sheathing device
- 300: Opening
- 301: Inner wall
- α: Angle
- A: Placement direction
- L: Longitudinal axis
- LA: Left atrium
- LV: Left ventricle
- M: Intra-cardiac tissue (myocardium)
- r, r1-r4: Radius of curvature
- RA: Right atrium
- RV: Right ventricle
- t: Tangent

## Claims

1. An implantable medical device (1), comprising:
- a housing (10) having a proximal end (101) and a distal end (100),
- an electrode device (11) arranged in the region of the distal end (100), and
- an anchoring device (2) fixedly attached to the housing (10) in the region of the distal end (100), the anchoring device (2) comprising at least one anchoring member (20) having a preshaped first configuration, the at least one anchoring member (20) being deflectable from the preshaped first configuration into a strained second configuration for placement of the implantable medical device (1) on an object,
wherein the at least one anchoring member (20) comprises a tip section (21) and a connection section (22) extending in between the tip section (21) and the distal end (100) of the housing (10), wherein at least in the preshaped first configuration the tip section (21) comprises a straight portion (212) adjoining the connection section (22) at a transition location (211) by forming a bend in between the straight portion (212) and the connection section (22).

2. The implantable medical device (1) according to claim 1, wherein at least in the preshaped first configuration the connection section (22) comprises at least one of at least one curved portion (224, 224A, 224A, 224C, 226) and at least one straight portion (222).

3. The implantable medical device (1) according to claim 1, wherein at least in the preshaped first configuration the connection section (22) comprises a multiplicity of curved portions (224, 224A, 224B, 224C, 226).

4. The implantable medical device (1) according to claim 3, wherein at least in the preshaped first configuration the curved portions (224A, 224B, 224C) have different radii of curvature (r1-r3).

5. The implantable medical device (1) according to claim 3, wherein at least in the preshaped first configuration a first of the curved portions (224, 226) comprises a first curvature and a second of the curved portions (224, 226) comprises a second curvature opposite the first curvature.

6. The implantable medical device (1) according to claim 3, wherein at least in the preshaped first configuration a straight portion (222) is arranged in between two of the curved portions (224, 224A, 224A, 224C, 226).

7. The implantable medical device (1) according to claim 1, wherein at least in the preshaped first configuration said straight portion (212) of the tip section (21) forms an angle (a) with a straight portion (222) of the connection section (22) or with a tangent (t) to a curved portion (224, 224C, 226) of the connection section (22).

8. The implantable medical device (1) according to claim 1, wherein the anchoring device (2) comprises a ring element (23) for fixing the anchoring device (2) to the housing (10) of the implantable medical device (1).

9. The implantable medical device (1) according to claim 1, wherein the at least one anchoring member (20) is made of a wire, a sheet, or a tube.

10. The implantable medical device (1) according to claim 1, wherein the at least one anchoring member (20) is made of an elastic metal material, in particular a Nickel Titanium alloy.

11. Delivery arrangement, comprising:
- a delivery system (3) having a sheathing device (30), and
- an implantable medical device (1) comprising a housing (10) having a proximal end (101) and a distal end (100), an electrode device (11) arranged in the region of the distal end (100), and an anchoring device (2) fixedly attached to the housing (10) in the region of the distal end (100), the anchoring device (2) comprising at least one anchoring member (20) having a preshaped first configuration, the at least one anchoring member (20) being deflectable from the preshaped first configuration into a strained second configuration for placement of the implantable medical device (1) on an object, wherein the at least one anchoring member (20) comprises a tip section (21) and a connection section (22) extending in between the tip section (21) and the distal end (100) of the housing (10), wherein at least in the preshaped first configuration the tip section (21) comprises a straight portion (212) adjoining the connection section (22) at a transition location (211) by forming a bend in between the straight portion (212) and the connection section (22),
wherein the at least one anchoring member (20), for placement of the implantable medical device (1) on an object by means of the delivery system (3), is received in the sheathing device (30) of the delivery system (3) in said strained second configuration.

12. The delivery arrangement according to claim 11, wherein the implantable medical device (1) generally extends along a longitudinal axis (L), the proximal end (101) and the distal end (100) being displaced with respect to each other along the longitudinal axis (L), wherein the implantable medical device (1) is deliverable from the sheathing device (30) for placement on said object in a placement direction (A) aligned with the longitudinal axis (L).

13. The delivery arrangement according to claim 12, wherein in the strained second configuration the straight portion (212) of the tip section (21) extends substantially parallel to the longitudinal axis (L) and points in the placement direction (A).

14. The delivery arrangement according to claim 12, wherein in the preshaped first configuration the straight portion (212) extends substantially parallel or at an obtuse angle with respect to the longitudinal axis (L).

15. The delivery arrangement according to claim 12, wherein in the preshaped first configuration the straight portion (212) points in a direction generally opposite to the placement direction (A).
